# EUROPÄISCHE PATENTSCHRIFT

(11) **EP 0 646 563 B1**
(45) Veröffentlichungstag und Bekanntmachung des Hinweises auf die Patenterteilung: **09.12.1998**
(21) Anmeldenummer: 94115122.7
(22) Anmeldetag: 26.09.1994
(51) Int. Cl.: C07C 29/17, C07C 45/50, C07C 45/72, C07C 69/80, C08K 5/12

(54) **Decylalkoholgemische, daraus erhältliche Phthalsäureester und ihre Verwendung als Weichmacher**
Decyl alcohol mixtures, phthalate esters obtained therefrom and their use as plasticisers
Mélanges d'alcools décyliques, esters de l'acide phtalique obtenus de ceux-ci et leur utilisation comme agents plastifiants

(30) Priorität: 30.09.1993 DE 4333324
(43) Veröffentlichungstag der Anmeldung: 05.04.1995
(73) Patentinhaber: Celanese GmbH, 60439 Frankfurt (DE)
(72) Erfinder: Bahrmann, Helmut, Dr., D-46499 Hamminkeln (DE); Greb, Wolfgang, Dr., D-46535 Dinslaken (DE); Heymanns, Peter, Dr., D-45147 Essen (DE); Lappe, Peter, Dr., D-46539 Dinslaken (DE); Müller, Thomas, D-46535 Dinslaken (DE); Szameitat, Jürgen, Dr., D-46487 Wesel (DE); Wiebus, Ernst, D-46147 Oberhausen (DE)

(56) Entgegenhaltungen:
- EP-A- 0 094 456
- EP-A- 0 562 451
- DE-A- 3 232 557
- DE-B- 1 165 568
- GB-A- 614 010

## Beschreibung

Die Erfindung betrifft Gemische isomerer Decylalkohole, ein Verfahren zu ihrer Herstellung, die aus diesen Alkoholen erhaltenen Phthalsäureester und deren Verwendung als Weichmacher.

Ester der Phthalsäure finden in großem Umfang als Weichmacher, insbesondere für Polyvinylchlorid, Verwendung. Als Alkoholkomponente werden vorwiegend primäre aliphatische Alkohole mit 8 bis 10 Kohlenstoffatomen eingesetzt, größte Bedeutung unter ihnen hat gegenwärtig das 2-Ethylhexanol. Phthalsäureester von Alkoholen mit weniger als 8 Kohlenstoffatomen im Molekül führen zu Weichmachern mit guter Gelierkraft. Nachteilig ist jedoch ihre höhere Flüchtigkeit. Phthalsäureester, die sich von primären aliphatischen Alkoholen mit mehr als 10 Kohlenstoffatomen ableiten, gelieren dagegen langsamer und sind weniger kaltebeständig.

Die Eigenschaften der Phthalsäureester-Weichmacher werden außer durch die Größe des Alkoholmoleküls auch durch die Verzweigung der Kohlenstoffkette beeinflußt. So ergeben wenig verzweigte Alkohole Esterweichmacher hoher Kälteflexibilität. Weitgehend lineare Alkohole mit 8 bis 10 Kohlenstoffatomen im Molekül gewinnen daher als Alkoholkomponente der Phthalsäureester zunehmend an Bedeutung. Voraussetzung für ihren Einsatz ist, daß sie in großen Mengen und kostengünstig zur Verfügung stehen.

Nach der deutschen Patentschrift 2 855 421 werden als Weichmacher Phthalate von C₉-Alkoholen eingesetzt, die durch Hydroformylierung von C₈-Olefinen, Hydrierung des Reaktionsproduktes und Veresterung der C₉-Alkohole mit Phthalsäureanhyrid erhalten werden. 3 bis 20 Gew.-% der Ausgangsolefine sollen in jeder Molekülkette ein Isobutanskelett, weniger als 3 Gew.-% der Olefine quaternären Kohlenstoff aufweisen und mehr als 90 Gew.-% der Gesamtmenge der Olefine als n-Octene, Monomethylheptene und Dimethylhexene vorliegen. Ferner soll das Gewichtsverhältnis der Gesamtmenge der n-Octene und Monomethylheptene zu den Dimethylhexenen mehr als 0,8 betragen.

Phthalsäureester von C₁₀-Alkoholen sind Gegenstand der europäischen Patentanmeldung 0 366 089. Die Alkohole werden zur Veresterung in Form eines Gemisches eingesetzt, das man durch Hydroformylierung einer Rutenfraktion, Aldolkondensation des erhaltenen Aldehydgemisches und anschließende Hydrierung gewinnt. Der Hydroformylierungsschritt unterliegt nach der Verfahrensbeschreibung keinen Beschränkungen. So kann als Katalysator sowohl Kobalt als auch Rhodium verwendet werden, der Zusatz einer organischen Verbindung des dreiwertigen Phosphors wird nicht ausgeschlossen.

Ein anderer Weg zur Gewinnung von Didecylphthalat-Gemischen ist in der europäischen Patentanmeldung 0 424 767 beschrieben. Die Herstellung der Ester erfolgt nach einem mehrstufigen Verfahren durch Dimerisierung von Butengemischen, Hydroformylierung und Hydrierung des resultierenden Octengemisches zu einem Nonanolgemisch, Dehydratisierung des Nonanolgemisches unter Bildung eines Nonengemisches und Hydroformylierung und Hydrierung des Nonengemisches unter Bildung eines Decanolgemisches.

In der EP-A-0 562 451 ist ferner ein Verfahren zur Herstellung eines Gemisches isomerer Decylalkohole beschrieben, welches unter Einbeziehung einer zweistufigen Hydroformylierung von Butenen abläuft. Diese zweistufige Hydroformylierung erfolgt in der 1. Stufe in einem wäßrigen/organischen Zweiphasensystem unter Verwendung eines wasserlöslichen Rhodium/Phosphin-Katalysators. In der 2. Stufe wird die Hydroformylierung dagegen in einem homogenen organischen System in Gegenwart eines wasserunlöslichen Rhodium-Katalysators durchgeführt.

Die bekannten Verfahren erfüllen in wirtschaftlicher und technischer Hinsicht noch nicht alle Anforderungen, die an einen im technischen Maßstab ausgeübten Prozeß gestellt werden, sei es, daß die Ausgangsstoffe nicht in ausreichender Menge und/oder nicht kostengünstig zur Verfügung stehen oder daß die Umwandlung der Ausgangsstoffe in die Alkohole an zu aufwendige Prozesse gebunden ist. Bei Mehrstufenverfahren, die die Hydroformylierung von Butenen einschließen, soll insbesondere der n-Valeraldehyd-Gehalt des Hydroformylierungsproduktes möglichst hoch sein, um die Bildung geradkettiger oder nur wenig verzweigter Alkohole zu fördern.

Es bestand daher die Aufgabe, ein Verfahren zu entwickeln, das nicht nur von Rohstoffen ausgeht, die preiswert zur Verfügung stehen, sondern die auch in technisch einfacher Weise in die gewünschten geradkettigen oder wenig verzweigten Alkohole überführt werden können.

Die Erfindung besteht in einem Verfahren zur Herstellung von Gemischen isomerer Decylalkohole, umfassend die Hydroformylierung von Buten-1 und Buten-2 enthaltenden Gemischen in zwei Stufen, wobei die Umsetzung in der ersten Stufe in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa und in der zweiten Stufe in homogener Phase in Gegenwart von Kobaltverbindungen als Katalysatoren bei Temperaturen von 130 bis 180°C und Drücken von 8 bis 30 MPa zu Aldehydgemischen erfolgt, Abtrennung und Vereinigung der erhaltenen Aldehydgemische aus den Hydroformylierungsstufen, Kondensation des vereinigten Aldehydgemisches unter Bildung eines Aldolgemischs und Abtrennung und Hydrierung des Aldolgemischs zu einem Gemisch isomerer Decylalkohole.

Die zur Herstellung des erfindungsgemäßen Gemischs isomerer Decylalkohole eingesetzten Buten-1 und Buten-2 enthaltenden Gemische fallen als Raffinerienebenprodukte bei der Herstellung von Automobiltreibstoffen und bei der Herstellung von Ethylen durch thermische Spaltung höherer Kohlenwasserstoffe zwangsweise in erheblichen Mengen an. Man gewinnt sie aus den C₄-Crackschnitten des Pyrolyseproduktes durch Extraktion des Butadiens mit einem selektiven Lösungsmittel und anschließende Abtrennung des Isobutens vorzugsweise durch Umwandlung in Methyltert.butylether. Das von Butadien befreite Pyrolyseprodukt wird als Raffinat I bezeichnet. Ist darüberhinaus auch noch das Isobuten abgetrennt, spricht man von Raffinat II. Statt das Butadien zu extrahieren, kann es auch im C₄-Crackschnitt partiell zu Butenen hydriert werden. Nach Abtrennung des i-Butens erhält man ein Buten-1/Buten-2-Gemisch, das besonders geeignet zur Weiterverarbeitung auf C₁₀-Alkohole ist. Schließlich geht man in letzter Zeit auch dazu über, das abgetrennte Butadien zu Butan zu hydrieren und in die Spaltung zurückzuführen um die Ethylen- und Propylenausbeute zu erhöhen.

Erfindungsgemäß werden Buten-1 und Buten-2 enthaltende Gemische z.B. in Form des Raffinats II, aber auch anderer Herkunft und Zusammensetzung in zwei Stufen hydroformyliert. In der ersten Stufe erfolgt bevorzugt die Umsetzung des Buten-1 zu einem Gemisch, das vorwiegend aus n-Valeraldehyd und, in untergeordneter Menge, aus i-Valeraldehyd besteht. Die Reaktion verläuft unter Bedingungen, die eine Isomerisierung des Buten-1 zu Buten-2 weitgehend ausschließen. In der zweiten Stufe wird das Buten-2 zu einem Gemisch von n-Valeraldehyd und i-Valeraldehyd hydroformyliert.

Die erste Stufe der Hydroformylierung führt man als heterogene Reaktion in einem Zweiphasensystem durch, eine Umsetzung, die z.B. in der DE-PS 26 27 354 beschrieben ist. Dieser Prozeß ist charakterisiert durch das Vorliegen einer organischen Phase, die die Ausgangsolefine und das Reaktionsprodukt enthält und einer wäßrigen Phase, in der der Katalysator gelöst ist. Als Katalysatoren werden wasserlösliche Rhodiumkomplexverbindungen eingesetzt, die wasserlösliche Phosphine als Liganden enthalten. Zu den Phosphinen zählen insbesondere Triarylphosphine, Trialkylphosphine und arylierte bzw. alkylierte Diphosphine, deren organische Reste durch Sulfonsäuregruppen oder Carboxylgruppen substituiert sind. Ihre Herstellung ist z.B. aus DE-PS 26 27 354 und DD-PS 259 194 bekannt. Die Reaktion der Butene erfolgt bei Temperaturen von 70 bis 150°C, vorzugsweise 100 bis 130°C und unter Drücken im Bereich von 0,4 bis 30, insbesondere 1 bis 10 MPa mit Wassergas, das Kohlenmonoxid und Wasserstoff im Volumenverhältnis 1 : 10 bis 10 : 1 enthält. Die Rhodiumkonzentration beträgt 20 bis 1000 Gew.-ppm, vorzugsweise 50 bis 500 Gew.-ppm, bezogen auf die wäßrige Katalysatorlösung. Je mol Rhodium setzt man 4 bis 100 mol wasserlösliches Phosphin ein. Das Volumenverhältnis von wäßriger zu organischer Phase beträgt 0,1 bis 10 : 1.

Der Butenumsatz je Zeiteinheit wird deutlich erhöht, wenn man der wäßrigen Katalysatorlösung ein Phasentransferreagenz (Lösungsvermittler) zusetzt. Es verändert die physikalischen Eigenschaften der Grenzflächen zwischen den beiden flüssigen Phasen und erleichtert den Übergang des organischen Reaktanten in die wäßrige Katalysatorphase.

Als Lösungsvermittler sind Verbindungen bekannt, deren hydrophile Gruppen ionisch (anionisch oder kationisch) oder nichtionisch sind. Zu den anionenaktiven Verbindungen gehören Natrium-, Kalium- oder Ammoniumsalze von Carbonsäuren mit 8 bis 20 Kohlenstoffatomen, insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagentien können in wäßriger Lösung nicht in Ionen dissoziieren. Zu ihnen zahlen Alkylpolyethylenglykole, Alkylphenylpolyethylenglykole, Fettsäurealkylolamide und Trialkylaminoxide. Schließlich finden auch Ampholyte wie Aminocarbonsäuren, Betaine und Sulfobetaine als Lösungsvermittler Anwendung.

Bewährt haben sich insbesondere kationische Lösungsvermittler der allgemeinen Formel [A-N(R¹R²R³)]⁺E⁻, in der A für einen geradkettigen oder verzweigten Alkylrest mit 6 bis 25 Kohlenstoffatomen steht, R¹, R², R³ gleich oder verschieden sind und geradkettige oder verzweigte Alkylreste mit 1 bis 5 Kohlenstoffatomen bedeuten und E für ein Anion, insbesondere für Sulfat, Tetrafluorborat, Acetat, Methosulfat, Benzolsulfonat, Alkylbenzolsulfonat, Toluolsulfonat, Lactat oder Citrat steht.

In der ersten Reaktionsstufe wird eine möglichst weitgehende Hydroformylierung des im Butengemisch enthaltenen Buten-1 zu n-Valeraldehyd angestrebt. Maßgebendes Kriterium für den Abbruch der Reaktion ist das vermehrte Auftreten von i-Valeraldehyd. Sein Anteil soll weniger als 10 Gew.-% (bezogen auf das Aldehydgemisch) betragen. So enthält das Aldehydgemisch bei einem Buten-1-Umsatz, der je nach den gewählten Reaktionsparametern bis zu 95 % beträgt, 90 und mehr Prozent n-Valeraldehyd, der Rest ist i-Valeraldehyd.

In der ersten Stufe nicht umgesetztes Olefin, vorwiegend Buten-2, verläßt zusammen mit Kohlenmonoxid, Wasserstoff und durch Hydrierung der Olefine entstandenem Butan den Reaktor. Das Gasgemisch wird ohne weitere Zwischenbehandlung auf 8 bis 30 MPa Druck komprimiert und bei Temperaturen von 130 bis 180°C in einer zweiten Reaktionsstufe in homogener Phase weiter umgesetzt. Als Katalysator findet Kobalt Anwendung. Es wird dem Reaktionsgemisch als Metall, zweckmäßig in feinverteilter Form oder, besser, als in organischen Medien lösliche Verbindung zugeführt, z.B. als Kobaltcarbonyl oder als Salz einer Carbonsäure, wie der 2-Ethylhexansäure. Die Kobaltkonzentration beträgt 0,1 bis 3 Gew.-%, vorzugsweise 0,6 bis 1,0 Gew.-%, bezogen auf die in die zweite Reaktionsstufe eingeleiteten Butene. Die Anwesenheit eines Lösungsmittels wie Toluol, Xylol oder Tetrahydrofuran ist nicht unbedingt erforderlich, weil seine Funktion vom Einsatzstoff und vom Reaktionsprodukt übernommen werden kann. Das Wassergas hat die gleiche Zusammensetzung wie im ersten Reaktionsschritt. In Abhängigkeit von den Reaktionsbedingungen werden bis zu 99 % des eingesetzen Olefins in n- und i-Valeraldehyd überführt.

Nach Beendigung der Hydroformylierung wird das Aldehydgemisch beider Reaktionsschritte vom Katalysator, von den nichtumgesetzten Reaktionsteilnehmern und den übrigen Reaktionsprodukten abgetrennt. Das geschieht im Falle der heterogenen Reaktion (erste Stufe) durch einfache Phasentrennung. Bei Umsetzung in homogener Phase, d.h. der zweiten Stufe des neuen Prozesses wird aus dem entspannten oder teilweise entspannten Reaktionsprodukt nach bekannten Verfahren, z.B. durch Behandlung mit Dampf (vgl. DE-C-3 032 252), der Katalysator entfernt.

Die Aldolkondensation der vereinigten Aldehydgemische der ersten und der zweiten Hydroformylierungsstufe erfolgt auf konventionellem Wege unter der Einwirkung basischer Katalysatoren. Eine Vorbehandlung der Aldehyde, z.B. eine spezielle Reinigung, ist nicht erforderlich. Als Katalysatoren finden Alkalicarbonate oder Alkalihydroxide, insbesondere Verbindungen des Natriums oder Kaliums und Amine, vorzugsweise tertiäre Amine, wie Triethylamin, Tri-n-propylamin, Tri-n-butylamin, Anwendung. Man arbeitet bei Temperaturen von 60 bis 160°C, insbesondere 80 bis 130°C und bei Normaldruck oder bei bis etwa 1 MPa erhöhtem Druck. Die Reaktionszeit beträgt wenige Minuten bis zu mehreren Stunden und ist insbesondere abhängig von Katalysatortyp und Reaktionstemperatur. Auf Grund seiner höheren Reaktionsgeschwindigkeit aldolisiert vornehmlich n-Valeraldehyd mit sich selbst oder mit isomeren Valeraldehyden zu Decenalen, eine Kondensation von 2-Methylvaleraldehyd oder Isovaleraldehyd untereinander tritt dagegen völlig in den Hintergrund.

Das durch Kondensation erhaltene Aldehydgemisch wird anschließend zu einem Decylalkoholgemisch hydriert. Die Wasserstoffanlagerung erfolgt in bekannter Weise in Gegenwart von Katalysatoren. Geeignet sind z.B. Hydrierkatalysatoren auf Basis von Nickel, Chrom oder Kupfer. Üblicherweise liegt die Hydriertemperatur zwischen 100 und 180°C und der Druck zwischen 1 und 10 MPa. Zur Reinigung wird das Decylalkoholgemisch destilliert. Es eignet sich vorzüglich als Alkoholkomponente in Phthalsäureestern, die als Weichmacher verwendet werden sollen.

Die Herstellung der Phthalsäureester ist bekannt [vgl. Ullmann, Encyclopädie der Technischen Chemie (1979), Bd. 18, Seite 536 ff]. Zweckmäßig setzt man Phthalsäureanhydrid mit dem Decylalkoholgemisch im Molverhältnis 1 : 2 in einer Stufe um. Die Reaktionsgeschwindigkeit kann durch Katalysatoren und/oder durch Erhöhung der Reaktionstemperatur erhöht werden. Um das Gleichgewicht in Richtung der Esterbildung zu verschieben, ist es erforderlich, das gebildete Wasser aus dem Reaktionsgemisch zu entfernen.

Die aus dem erfindungsgemäßen Decylalkoholgemisch erhaltenen Phthalate zeichnen sich als Weichmacher durch hervorragende Kälteeigenschaften aus.

## Patentansprüche

1. Verfahren zur Herstellung von Gemischen isomerer Decylalkohole, umfassend die Hydroformylierung von Buten-1 und Buten-2 enthaltenden Gemischen in zwei Stufen, wobei die Umsetzung in der ersten Stufe in einem heterogenen Reaktionssystem unter Verwendung wasserlösliche Phosphine in komplexer Bindung enthaltender Rhodiumverbindungen als Katalysatoren bei Temperaturen von 70 bis 150°C und Drücken von 0,4 bis 30 MPa und in der zweiten Stufe in homogener Phase in Gegenwart von Kobaltverbindungen als Katalysatoren bei Temperaturen von 130-180°C und Drücken von 8 bis 30 MPa zu Aldehydgemischen erfolgt, Abtrennung und Vereinigung der erhaltenen Aldehydgemische aus den Hydroformylierungsstufen, Kondensation des vereinigten Aldehydgemischs unter Bildung eines Aldolgemischs und Abtrennung und Hydrierung des Aldolgemischs zu einem Gemisch isomerer Decylalkohole.

2. Verfahren zur Herstellung von Gemischen isomerer Decylalkohole nach Anspruch 1, dadurch gekennzeichnet, daß die Umsetzung in der ersten Hydroformylierungsstufe bei 100 bis 130°C und unter Drücken von 1 bis 10 MPa erfolgt.

3. Verfahren zur Herstellung von Gemischen isomerer Decylalkohole nach Anspruch 1 oder 2, dadurch gekennzeichnet, daß die Umsetzung in der ersten Hydroformylierungsstufe bei einer Rhodiumkonzentration von 20 bis 1000 Gew.-ppm, vorzugsweise 50 bis 500 Gew.-ppm, bezogen auf die wäßrige Katalysatorlösung, erfolgt.

4. Verfahren zur Herstellung von Gemischen isomerer Decylalkohole nach einem oder mehreren der Ansprüche 1 bis 3, dadurch gekennzeichnet, daß in der ersten Hydroformylierungsstufe je mol Rhodium 4 bis 100 mol wasserlösliches Phosphin eingesetzt werden.

5. Verfahren zur Herstellung von Gemischen isomerer Decylalkohole nach einem oder mehreren der Ansprüche 1 bis 4, dadurch gekennzeichnet, daß in der ersten Hydroformylierungsstufe der wäßrigen Katalysatorlösung ein Phasentransferreagenz zugesetzt wird.

6. Verfahren zur Herstellung von Gemischen isomerer Decylalkohole nach einem oder mehreren der Ansprüche 1 bis 5, dadurch gekennzeichnet, daß die Umsetzung in der zweiten Hydroformylierungsstufe bei einer Kobaltkonzentration von 0,1 bis 3 Gew.-%, vorzugsweise 0,6 bis 1 Gew.-%, bezogen auf die in die zweite Reaktionsstufe eingeleiteten Butene, erfolgt.

## Claims

1. A process for preparing mixtures of isomeric decyl alcohols, comprising the hydroformylation of mixtures containing butene-1 and butene-2 in two stages, the reaction in the first stage taking place in a heterogeneous reaction system with the use of rhodium compounds containing water-soluble phosphines in a complex bond as catalysts at temperatures of 70 to 150°C and at pressures of 0.4 to 30 MPa and in the second stage in a homogeneous phase in the presence of cobalt compounds as catalysts at temperatures of 130 to 180°C and at pressures of 8 to 30 MPa to form aldehyde mixtures, separation and combination of the aldehyde mixtures obtained from the hydroformylation stages condensation of the combined aldehyde mixture with the formation of an aldol mixture and separation and hydrogenation of the aldol mixture to form a mixture of isomeric decyl alcohols.

2. The process for preparing mixtures of isomeric decyl alcohols according to claim 1, characterised in that the reaction in the first hydroformylation stage takes place at 100 to 130°C and at pressures of 1 to 10 MPa.

3. The process for preparing mixtures of isomeric decyl alcohols according to claim 1 or 2, characterised in that the reaction in the first hydroformylation stage takes place with a rhodium concentration of 20 to 1000 ppm by weight, preferably 50 to 500 ppm by weight, related to the aqueous catalyst solution.

4. The process for preparing mixtures of isomeric decyl alcohols according to one or more of claims 1 to 3, characterised in that 4 to 100 mol of water-soluble phosphine are used per mole of rhodium in the first hydroformylation stage.

5. The process for preparing mixtures of isomeric decyl alcohols according to one or more of claims 1 to 4, characterised in that a phase transfer reagent is added to the aqueous catalyst solution in the first hydroformylation stage.

6. The process for preparing mixtures of isomeric decyl alcohols according to one or more of claims 1 to 5, characterised in that the reaction in the second hydroformylation stage takes place with a cobalt concentration of 0.1 to 3% by weight, preferably 0.6 to 1% by weight, related to the butenes introduced into the second reaction stage.

## Revendications

1. Procédé de préparation de mélanges d'alcools décyliques isomères, englobant l'hydroformylation de mélanges contenant le 1-butène et le 2-butène, en deux étapes, la réaction conduisant à des mélanges d'aldéhydes ayant lieu dans la première étape dans un système réactionnel hétérogène en utilisant comme catalyseurs des composés hydrosolubles de rhodium contenant de la phosphine en liaison complexe, à des températures de 70 à 150°C et des pressions de 0,4 à 30 MPa et dans la seconde étape dans une phase homogène de composés de cobalt comme catalyseurs à des températures de 130 à 180°C et des pressions de 8 à 30 Mpa, la séparation et la purification des mélanges d'aldéhydes obtenus dans les étapes d'hydroformylation, la condensation du mélange d'aldéhydes purifié avec formation d'un mélange d'aldols et la séparation et l'hydrogénation du mélange d'aldols en un mélange d'alcools décyliques isomères.

2. Procédé de préparation de mélanges d'alcools décyliques isomères selon la revendication 1, caractérisé en ce que la réaction de la première étape d'hydroformylation a lieu de 100 à 130°C et sous des pressions de 1 à 10 MPa.

3. Procédé de préparation de mélanges d'alcools décyliques isomères selon la revendication 1 ou 2, caractérisé en ce que la réaction de la première étape d'hydroformylation a lieu à une concentration de rhodium de 20 à 1 000 ppm en poids, de préférence de 50 à 500 ppm en poids, par rapport à la solution aqueuse de catalyseur.

4. Procédé de préparation de mélanges d'alcools décyliques isomères selon l'une ou plusieurs des revendications 1 à 3, caractérisé en ce que dans la première étape d'hydroformylation chaque mole de rhodium est utilisée pour 4 à 100 moles de phosphine hydrosoluble.

5. Procédé de préparation de mélanges d'alcools décyliques isomères selon l'une ou plusieurs des revendications 1 à 4, caractérisé en ce que dans la première étape d'hydroformylation on ajoute un réactif transfert de phases à la solution aqueuse de catalyseur.

6. Procédé de préparation de mélanges d'alcools décyliques isomères selon l'une ou plusieurs des revendications 1 à 5, caractérisé en ce que la réaction dans la seconde étape d'hydroformylation a lieu à une concentration de cobalt de 0 1 à 3 % en poids, de préférence de 0,6 à 1 % en poids, par rapport au butène amené dans la seconde étape réactionnelle.
